Europäisches Patentamt

(19) European Patent Office

Office européen des brevets

(11) Numéro de publication: **0 233 265**
**B1**

(12) **FASCICULE DE BREVET EUROPEEN**

(45) Date de publication du fascicule du brevet: **31.10.90**

(21) Numéro de dépôt: **86905261.3**

(22) Date de dépôt: **08.08.86**

⑯ Numéro de dépót international:
**PCT/EP86/00470**

(87) Numéro de publication internationale:
**WO 87/01042 26.02.87 Gazette 87/05**

(51) Int. Cl.⁵: **A 61 M 5/31**

(54) **SERINGUE PREREMPLIE.**

(30) Priorité: **13.08.85 FR 8512441**

(43) Date de publication de la demande:
**26.08.87 Bulletin 87/35**

(45) Mention de la délivrance du brevet:
**31.10.90 Bulletin 90/44**

(84) Etats contractants désignés:
**AT BE CH DE FR GB IT LI LU NL SE**

(56) Documents cités:
**EP-A-0 115 931**
**EP-A-0 150 681**
**FR-A-2 024 089**
**FR-A-2 178 019**
**FR-A-2 529 086**
**US-A-2 453 590**
**US-A-3 943 927**
**US-A-4 091 812**
**US-A-4 227 528**

(73) Titulaire: **MEDICORP HOLDING S.A.**
**11 Boulevard du Prince Henri**
**L-2014 Luxembourg (LU)**

(72) Inventeur: **MEYER, Gabriel**
**10A, chemin des Princes**
**CH-1222 Vésenaz (CH)**
Inventeur: **HOWALD, Ernst**
**10B, chemin des Princes**
**CH-1222 Vésenaz (CH)**

(74) Mandataire: **Nithardt, Roland**
**CABINET ROLAND NITHARDT Attn: Cabinet**
**MOSER & CIE Rue Edouard Verdan 15**
**CH-1400 Yverdon-les-Bains (CH)**

Courier Press, Leamington Spa, England.

## Description

La présente invention concerne une seringue préremplie comportant un réservoir allongé ouvert à une de ses extrémités et pourvu d'un étranglement voisin de cette extrémité, ce réservoir contenant un liquide, notamment un médicament liquide à injecter, un organe d'injection mobile par rapport à ce réservoir, cet organe d'injection comprenant un organe d'obturation en élastomère au moins partiellement engagé dans le réservoir dans l'étranglement, et agencé pour obturer le réservoir lorsque la seringue est dans une première position dite de stockage, cet organe d'obturation étant équipé d'un conduit central composé d'une branche axiale et d'au moins une branche radiale pour mettre en communication une aiguille avec l'intérieur du réservoir de manière à permettre l'évacuation du liquide quand la seringue est dans une seconde position dite d'injection, la seringue comportant en outre une capsule solidaire de cet organe d'obturation et montée sur le réservoir de telle manière qu'un tronçon d'extrémité de ce réservoir, voisin de son ouverture, soit engagé dans cette capsule de telle manière que le déplacement relatif de la capsule et du réservoir engendre un déplacement équivalent de l'organe d'obturation dans le résrvoir suffisant pour refouler une quantité de médicament suffisante pour remplir totalement le conduit central et apparaître sous la forme d'une goutte à l'extrémité de l'aiguille.

Les seringues préremplies de ce type, décrites par exemple dans la publication européenne EP—A—150 681, sont conçues pour pouvoir être stockées pendant une période pouvant être relativement longue. A cet effet, l'organe d'obturation comporte une tête en élastomère qui est fortement comprimée à l'intérieur du col du réservoir. Cet organe d'obturation comporte par ailleurs des organes de retenue élastiques qui ont tendance à le retenir dans sa position et à empêcher tout déplacement vers l'intérieur du réservoir. Enfin, il règne à l'intérieur de ce réservoir une surpression qui oppose également une force tendant à empêcher la pénétration de l'organe d'obturation à l'intérieur du réservoir pendant toute la phase du stockage.

Il en résulte que pour emener l'organe d'obturtation de sa position de stockage dans laquelle il obture de façon étanche l'extrémité ouverte du réservoir, vers sa position d'injection dans laquelle il remplit le double rôle de piston et de vanne pour permettre l'évacuation du médicament liquide hors du réservoir, il est nécessaire d'exercer une certaine force susceptible de vaincre l'inertie initiale. Si cette force est exercée dans contrôle ni retenue, une partie du liquide contenu ans le réservoir risque d'être éjecté et perdu. Etant donné que le volume de médicament liquide contenu dans le réservoir peut correspondre à une dose unitaire, il est essentiel que cette dose soit injectée en totalité et que la perte de médicament due au phénomème mentionné ci-dessus soit évitée, surtout lorsqu'il s'agit de doses de faible volume.

La présente invention se propose de résoudre ce problème en prévoyant des moyens permettant de contrôler de façon précise le passage de la seringue de sa position de stockage à sa position d'injection, et en particulier d'effectuer le débullage de la seringue pour la rendre absolument prête à l'utilisation.

Dans ce but, la seringue préremplie selon l'invention est caractérisée en ce que la seringue est équipée d'un capuchon adapté par-dessus l'extrémité fermée du réservoir allongé, et en ce que ce capuchon est pourvu de moyens de liaison amovibles pour rendre ce capuchon solidaire de la capsule lorsque la seringue est dans sa première position et de moyens d'appui pour permettre un déplacement relatif limité de l'organe d'injection mobile et du réservoir pour emener la seringue préremplie de sa première position dans sa seconde position, lesdits moyens de liaison amovibles comportant une embase fixée à la capsule, cette embase étant liée au capuchon par une zone de moindre résistance.

Ladite embase est de préférence fixée à un rebord annulaire ménagé autour de la capsule au voisinage de son extrémité dans laquelle est engagée l'extrémité ouverte du réservoir.

Ledit rebord annulaire ménagé autour de la capsule forme de préférence deux ailettes latérales de maintien de la seringue.

Ladite embase est avantageusement soudée par ultrasons au rebord annulaire.

Lesdits moyens d'appui comprennent de préférence au moins une butée agencée pour coopérer avec un rebord du capuchon et la distance entre cette butée et ce rebord est de préférence sensiblement égale audit déplacement relatif limité de l'organe d'injection mobile et du réservoir. De ce fait, lorsque ces deux éléments sont effectivement déplacés l'un par rapport à l'autre de cette distance séparant la butée du rebord, la seringue est débullée, c'est-à-dire amenée dans sa position d'utilisation.

Selon un mode de réalisation préféré, ladite butée comprend une surface dudit rebord annulaire de ladite embase et ledit rebord du capuchon qui coopère avec cette butée est lié à cette embase par la zone de moindre résistance.

L'embase a de préférence une forme générale cylindrique ou conique dont le plus petit diamètre est supérieur au diamètre extérieur du capuchon de forme générale cylindrique.

La zone de moindre résistance est avantageusement constituée par un élément annulaire fermé dont la surface s'étend latéralement vers l'extérieur du capuchon.

Selon une autre mode de réalisation, la zone de moindre résistance est constituée par un ensemble de pattes dressées latéralement vers l'extérieur du capuchon.

Ce capuchon comporte de préférence au moins un organe d'appui ménagé à l'intérieur et conçu pour prendre appui contre le fond fermé du réservoir.

La présente invention sera mieux comprise en référence à la description d'un exemple de réalisation et du dessin annexé dans lequel:

La figure 1 représente une vue en coupe longitudinale de la seringue selon l'invention, telle qu'elle se présente dans sa position de stockage,

La figure 2 représente un vue partielle en coupe longitudinale de la seringue selon la figure 1 telle qu'elle se présente au moment où elle est amenée de sa position de stockage dans sa position d'injection, et

La figure 3 représente une vue partielle, en coupe longitudinale et agrandie d'un détail de la seringue des figures précédentes.

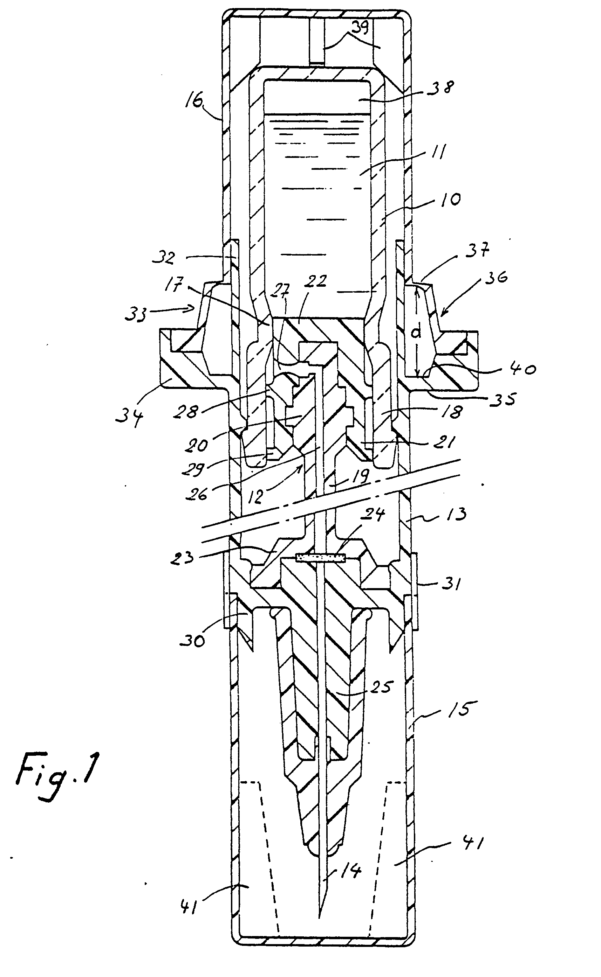

La figure 1 représente à titre d'exemple une seringue préremplie à dose unitaire qui comprend essentiellement un réservoir 10 contenant un médicament liquide 11 à injecter, un organe d'obturation 12, une capsule 13 rendue solidaire de l'organe d'obturation 12, une aiguille 14, un cache-aiguille 15 et un capuchon 16 servant à protéger le réservoir 10. On constate que par leur forme, le cache-aiguille 15 et le capuchon 16 coopérent avec la capsule 13 pour constituer une enceinte protectrice inviolable des parties fonctionnelles de la seringue. Le réservoir 10 comporte un étranglement 17 au voisinage de son col 18 dans lequel est engagé l'organe d'obturation 12. Ce dernier se compose essentiellement d'une tige de piston 19 dont une première extrémite élargie 20 sert d'organe de raidissement à une jupe annulaire 21 reliée à une tête de piston 22 qui joue effectivement le rôle d'organe d'obturation du réservoir pendant la période de stockage de la seringue. La seconde extrémité 23 de la tige de piston est évasée et comporte des organes de couplage pour fixer ladite tige de piston coaxialement à l'intérieur de la capsule 13. Un filtre 24 est avantageusement monté au fond de la capsule, dans un logement adéquat, pris entre la base de le tige de piston et l'embout porte-aiguille 25 solidaire de la capsule. Un conduit central composé d'une branche axiale 26 et d'une branche transversale 27 traverse successivement l'embout porte-aiguille, la tige de piston et la jupe annulaire de l'organe d'obturation pour mette en communication, au moment de l'injection, l'intérieure du réservoir avec l'aiguille d'injection.

La jupe annulaire 21 est équipée d'une lèvre annulaire 28 qui joue le rôle d'un segment de piston pendant la phase d'injection et qui contribue à assurer le maintien en position de l'organe d'oburation pendant la phase de stockage. Le col 18 et l'intérieur du réservoir 10 ont sensiblement le même diamètre. La lèvre annulaire 28 a un diamètre tel qu'elle soit faiblement comprimée pendant ladite phase de stockage, de manière à éviter les risques d'une déformation permanente de cette lèvre suite à une forte compression pendant une durée relativement longue.

La tête de piston 22 est dimensionnée de telle manière qu'elle soit fortement comprimée au niveau de l'étranglement 17 pour assurer une étanchéité parfaite du réservoir pendant la phase de stockage. Dans son état détendu, son diamètre est cependant inférieur au diamètre intérieur du réservoir pour permettre le passage du médicament à injecter vers la branche transversale 27 du conduit central pendant la phase d'injection.

Le long de son extrémité libre, la jupe annulaire 21 comporte un bourrelet 29 qui joue le rôle de butée d'arrêt en coopérant avec la rampe conique convergeant vers l'étranglement 17 et définissant la position exacte de l'organe d'oburation au moment de la phase de débullage précédant la phase d'injection.

Le cache-aiguille 15 est adapté sur un rebord 30 ménagé à la base de la capsule, coaxialement à l'embout porte-aiguille. Il est avantageusement rendu solidaire de cette capsule au moyen d'une étiquette 31, portant diverses inscriptions permettant notamment l'identification du médicament à injecter ou des formules d'utilisation et comportant une zone de moindre résistance au point de jonction entre le cache-aiguille et la capsule.

Le capuchon 16 est adapté sur une collerette 32 ménagée à l'extrémité de la capsule, coaxialement au réservoir. Il comporte des moyens de liaison amovibles 33 qui le rendent solidaire d'ailettes 34 qui sont elles-mêmes solidaires d'un rebord annulaire 35 ménagé autour de l'extrémité supérieure de la capsule 13. Ces moyens de liaison amovibles 33 comportent une embase 36 liée au capuchon 10 par l'intermédaire d'un élément annulaire 37 continu ou par l'intermédiare de pattes discontinues qui s'étendent s'étendent latéralement autour de ce capuchon et constituent une zone de moindre résistance susceptible d'être brisée par l'utilisateur. L'embase 36 a une forme générale cylindrique ou conique, dont le plus diamètre est supérieure au diamètre extérieur du capuchon 16 ayant de préférence une forme générale cylindrique. Cette embase présente avantageusement une forme qui lui permet de s'empoîter dans un évidemment approprié ménagé dans les ailettes 34 et d'être soudée, de préférence aux ultrasons, à ces ailettes.

L'étiquette 31 d'une part et les moyens de liaison amovibles 33 d'autre part garantissent l'inviolabilité et l'étanchéité de l'enceinte renfermant les éléments fonctionnels de la seringue pendant le stockage et constitué par la capsule 13 qui s'emboîte dans le cacheaiguille 15 et le capuchon 16.

L'utilisation de la seringue décrite ci-dessus nécessite une phase préparatoire très qui évite toute manipulation directe du medicament et, par conséquent, supprime totalement tout risque d'erreur et de contamination. Cette phase préparatoir consiste tout d'abord à retourner la seringue de telle manière que l'aiguille 14 pointe vers le hat et que le volume d'aire ou de gaz 38 surmontant le médicament liquide 11 contenu dans le réservoir 10 soit amené en contact avec la tête de piston 22. La seringue étant maintenue dans cette position, l'opérateur exerce une force axiale, de préférence au moyen du pouce, sur le fond du capuchon 16 tout en maintenant la seringue dans sa position, par ces ailettes 34, de préférence au moyen de l'index et du majeur de la même main. Cette force a tout d'abord comme effet de briser la liaison entre le capuchon 16 et l'embase 36 au niveau de la zone de moindre résistance définie par l'élément annulaire 37. Elle a ensuite pour conséquence un déplacement relatif du réservoir 10 et de l'organe d'obturation, ce déplacement relatif permettant d'amener l'ouverture de la branche transversale 27 du conduit central au-delà de l'étranglement 17 à l'intérrieur du réservoir 10. En effet, la capuchon 16 comporte des organes d'appui 39 ménagés sur son fond ou sur ses parois latérales intérieures qui sont agencés pour prendre appui contre la surface extérieure du fond du capuchon 16. De ce fait, le déplacement du capuchon 16 bar rapport à la capsule 13 induit un déplacement relatif du réservoir 10 et de l'organe d'obturation, le premier étant rendu solidaire du capuchon 16 par les éléments d'appui 39 et le second étant solidaire du capuchon 13. L'amplitude de ce déplacement relatif est égal à la distance d qui sépare l'extrémité du capuchon ou la zone de moindre résistance 37 ajacente à cette extrémité et la surface annulaire 40 du rebord annulaire 35 de la capsule 13.

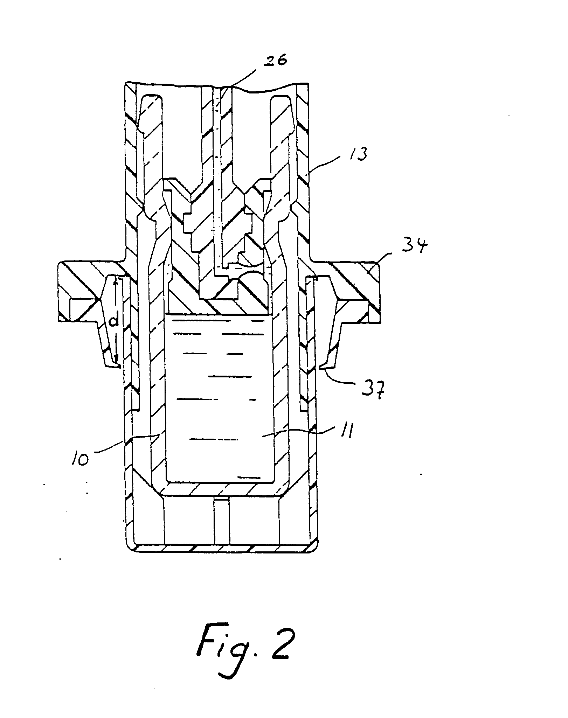

Cette position est représentée par la figure 2. La distance d est suffisante pour enfoncer la tête de piston 22 à l'intérieur du réservoir 10 pour d'une part évacuer en totalité le gaz 38 et d'autre part refouler une quantité de médicament liquide 11 suffisante pour remplir totalement le cond

A partir de ce moment l'utilisateur peut retirer le capuchon 16 et procéder à l'injection en enfonçant progressivement le réservoir 10 dans la capsule 13, ce qui a pour effet de faire pénétrer l'organe d'obturation 12 qui remplit à partir de ce moment le rôle conjugé d'un piston et d'une vanne, à l'intérieur de ce réservoir pour en refouler le contenu à travers le conduit central 26. Dans ce but, l'opérateur peut directement appuyer sur le fond du réservoir 10 par exemple à l'aide de son pouce en retenant la seringue par ses ailettes 34 au moyen de l'index et du majeur. Il peut également utiliser le cacheaiguille 15, éventuellement équipé de butées intérieurs 41, préalablement retiré de l'endroit où il est positionné pendant le phase de stockage et mis à la place du capuchon 16. Pour retirer le cacheaiguille 15, il est indispensable de déchirer l'étiquette de liaison 31 qui, comme mentionné précédemment, comporte de préférence une zone de moindre résistance constituée par exemple par des entailles discontinues disposées le long d'une ligne circulaire.

L'utilisation du cache-aiguille pour enfoncer le réservoir dans la capsule est particulièrement avantageux lorsque le réservoir comporte une double dose de médicament à injecter à des endroits différents ou à des moments différents. Dans ce cas, les butées 41 sont définies de telle manière que la première demi-dose est injectée lorsque l'extrémité libre du cache-aiguille 15 est amenée en appui contre la surface annulaire 40 et la seconde demi-dose est injectée lorsque le réservoir 10 est complétement enfoncé à l'intérieur de la capsule 13, c'est-à-dire lorsque la tête de piston 22 est en appui contre le fond du réservoir. Il est bien entendu que l'injection de la seconde demi-dose ne peut se faire qu'après le retrait préalable du cache-aiguille 15.

Comme mentionné précédemment, le capuchon 16 a pour but de vaincre les résistances et contraintes s'opposant initialement au déplacement de l'organe d'obturation vers l'intérieur du réservoir, sans risquer de faire gicler le médicament d'une manière incontrôlée hors de la seringue. L'inertie initiale est d'autant plus grande que le serrage de l'organe d'obturation contre les parois du réservoir et plus particulièrement contre les parois du col de ce réservoir est plus important. Or pour assurer une bonne étanchéité du réservoir pendant la phase de stockage qui peut être plus ou moins longue, on a intérêt à ce que ce serrage soit aussi important que possible. En outre, il règne à l'intérieur du réservoir une surpression particulièrement avantageuse pour la conservation du médicament. Cette surpression peut, dans certains cas, être exclusivement induite lors de la mise en place de l'organe d'obturation, qui a pour effet de réduire le volume intérieur du réservoir et, par conséquent, de comprimer l'atmosphère surmontant le liquide médicamenteux. Elle pout également, dans d'autres cas, être du à la somme de cette surpression induite et d'une surpression engendrée par des moyens techniques connus soit pendant, soit après le remplissage de la seringue. L'effet combiné de ces différentes contraites crée une résistance qu'il faut vaincre et que le dispositif permet de contrarier avec précisoin et sans risque.

L'utilisation de ce dispositif, tout en facilitant les manipulations, permet de normaliser le débullage en le rendant quasiment automatique et en garantissant son résultat. Par ailleurs, on peut affirmer qu'elle autorise indirectement le stockage du médicament en surpression dans le réservoir, ce qui pour conséquence d'améliorer l'étanchéité de ce réservoir en augmentant le serrage entre le col et l'organe d'obturation et, par suite, d'accroître la durée de conservation du médicament dans la seringue.

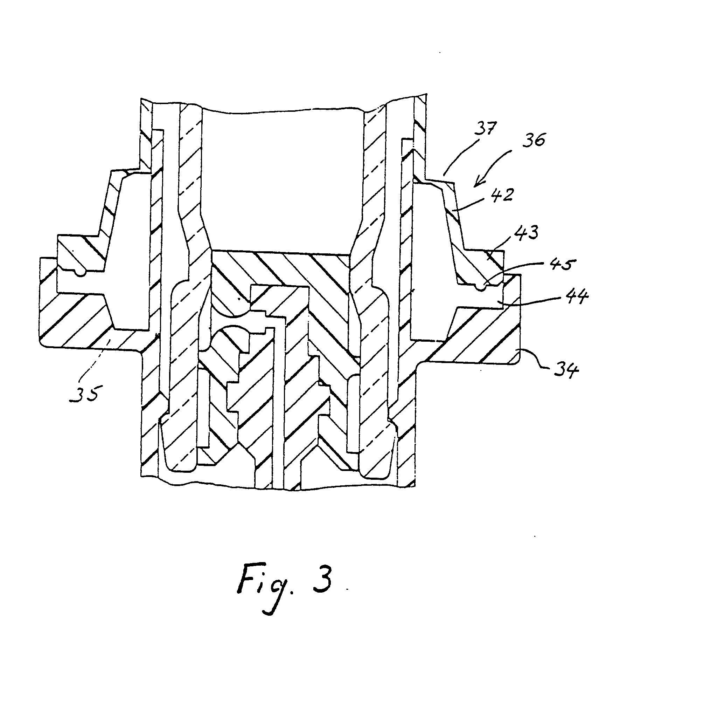

La figure 3 représente plus en détail l'embase 36 et son mode de liaison avec les ailettes 34. L'embase 36 se compose d'une zone centrale troncinique 42 qui relie entre elles d'une part l'élément annulaire 37 qui constitue la zone de moindre résistance et une semelle annulaire 43 agencée pour s'emboîter dans un évidement 44 ménagé dans le rebord annulaire portant les ailettes 34. La semelle annulaire 43 comporte une protubérance annulaire 45 qui est en fait un bourrelet de matière destiné à permettre la soudure aux ultrasons, par application d'une sonotrode, de l'embase et des ailettes. La soudure obtenue est suffisamment résistante pour que le rupture des moyens de liaison amovibles entre le capuchon 16 et la capsule 13 se fasse au niveau de la zone de moindre résistance et non au niveau de la zone soudée.

La présente invention n'est pas limitée aux formes de réalisation décrites et illustrées par les figures mais peut subir différentes modifications et se présenter sous diverses variantes évidentes pour l'homme de l'art.

## Revendications

1. Seringue préemplie comportant un réservoir (10) allongé ouvert à une de ses extrémités (18) et pourvu d'un étranglement (17) voisin de cette extrémité, ce réservoir (10) contenant un liquide (11), notamment un médicament liquide à injecter, un organe d'injection mobile par rapport à ce réservoir, cet organe d'injection compenant un organe d'obturation (12) en élastomère au moins partiellement engagé dans le réservoir (10) dans l'étranglement (17), et agencé pour obturer le réservoir (10) lorsque la seringue est dans une première position dite de stockage, cet organe d'obturation étant équipé d'un conduit central composé d'une branche axiale (26) et d'au moins une branche radiale (27) pour mettre en communication une aiguille (14) avec l'intérieur du réservoir (10) de manière à permettre l'évacuation du liquide quand la seringue est dans une seconde position dite d'injection, la seringue comportant en outre une capsule (13) solidaire de cet organe d'obturation (12) et montée sur le réservoir (10) de telle manière qu'un tronçon d'extrémité de ce réservoir, voisin de son ouverture (18), soit engagé dans cette capsule (13) de telle manière que le déplacement relatif de la capsule (13) et du réservoir (10) engendre un déplacement équivalent de l'organe d'obturation (12) dans le réservoir (10) suffisant pour refouler une quantité de médicament (11) suffisante pour remplir totalement le conduit central et apparaitre sous la forme d'une goutte à l'extrémité de l'aiguille (14), caractérise en ce que la seringue est équipée d'un capuchon (16) adapté par-dessus l'extrémité fermée du réservoir allongé, et en ce que ce capuchon (16) est pourvu de moyens de liaison amovibles (33) pour rendre ce capuchon solidaire de la capsule (13) lorsque la seringue est dans sa première position et de moyens d'appui (39, 40) pour permettre un déplacement relatif limité de l'organe d'injection mobile et du réservoir pour amener la seringue préemplie de sa première position dans sa seconde position, lesdites moyens de liaison amovibles (33) comportant une embase (36) fixée à la capsule (13), cette embase étant liée au capuchon (16) par une zone de moindre résistance.

2. Seringue selon la revendication 1, caractérisée en ce que ladite embase (36) est fixée à un rebord annlaire (35) ménagé autour de la capsule (13) au voisinage de son extrémité dans laquelle est engagée l'extrémité ouverte du reservoir.

3. Seringue selon la revendication 2, caractérisée en ce que ledit rebord annulaire (35) ménagé autour de la capsule (13) forme au moins deux ailettes latérales (34) de maintien de la seringue.

4. Seringue selon la revendication 2, caractérisée en ce que ladite embase (36) est soudée par ultrasons audit rebord annulaire (35).

5. Seringue selon la revendication 1, caractérisée en ce que lesdits moyens d'appui comprennent au moins une buteé (40) agencée pour copérer avec un rebord du capuchon (16) et en ce que la distance (d) entre cette butée et ce rebord est égale audit déplacement relatif limité de l'organe d'injection mobile et du réservoir.

6. Seringue selon la revendication 5, caractérisée en ce que ladite butée comprend une surface (40) dudit rebord annulaire (35) de ladite capsule (13) et en ce que ledit rebord du capuchon (16) qui coopère avec cette butée est lié à ladite embase (36) par une zone de moindre résistance.

7. Seringue selon la revendication 1, caractérisée en ce que l'embase a une forme générale cylindrique conique dont le plus petit diamètre est supérieur au diamètre extérieur du capuchon (16) de forme générale cylindrique.

8. Seringue selon la revendication 7, caractérisée en ce que la zone de moindre résistance est constituée par un élément annulaire (37) fermé dont la surface s'étend latéralement vedrs l'extérieur du capuchon (16).

9. Seringue selon la revendication 7, caractérisée en ce que la zone de moindre résistance est constituée par un ensemble de pattes dressées latéralement vers l'extérieur du capuchon.

10. Seringue selon la revendication 1, caractérisée en ce que le capuchon comporte au moins un organe d'appui ménagé à l'intérieur et conçu pour prendre appui contre le fond fermé du réservoir (10).

11. Seringue selon la revendication 1, comportant un cache-aiguille (15), caractérisée en ce que ce cache-aiguille est pourvu de butées (41) agencées pour servir de limiteur de course conçu pour contrôler le déplacement relatif du réservoir (10) et de l'organe d'obturation (12), ces butées étant agencées pour que ce déplacement relatif corresponde à l'injection d'une demi-dose de médicament.

## Patentansprüche

1. Vorgefüllte Spritze, bestehend aus einem langgezogenen Behälter (10), der an einem seiner Enden (18) offen und in der Nähe dieses Endes mit einer Einschnürung versehen ist, wobeï dieser Behälter (10) eine Flüssigkeit (11), insbesondere eine flüssige Arznei, die einzuspritzen ist, enthält, und aus einem Einspritzteil besteht, der gegenbüber diesem Behälter beweglich ist, wobei dieser Einspritzteil einen Verschlußteil (12) aus Elastomer enthält, der mindestens teilweise in die Einschnürung (17) des Behälters (10) eingeführt und so ausgebildet ist, um den Behälter (10) zu verschließen, wenn die Spritze sich in einer ersten Stellung, genannt Lagerstellung befindet, wobei dieser Verschlußteil mit einer zentralen Leitung versehen ist, die aus einem axialen Schenkel (26) und mindestens aus einem radialen Schenkel (27) besteht, um eine Nadel (14) mit dem Inneren des Behälters in Verbindung zu bringen, damit die Flüssigkeit abfließen kann, wenn die Spritze sich in einer zweiten Stellung, genannt Einspritzstellung, befindet, wobei die Spritze außerdem aus einer Kapsel (13) besteht, die mit diesem Verschlußteil (12) fest verbunden und auf dem Behälter (10) derart aufgesetzt ist, daß ein Teil des Endes dieses Behälters in der Nähe seiner Öffnung (18) in diese Kapsel derart eingeführt ist, daß die relative Verstellung der Kapsel (13) und des Behälters (10) eine gleichwertige Verstellung des Verschlußteils (12) in dem Behälter (10) bewirkt, die ausreicht, um eine ausreichende Menge der Arznei (11) zu verdrängen, damit die zentrale Leitung völlig gefüllt ist, und in der Form eines Tropfens am Ende der Nadel heraustritt, dadurch gekennzeichnet, daß die Spritze mit einer Kappe (16) versehen ist, die auf dem geschlossenen Ende des langgezogenen Behälters angebracht ist, und daß die Kappe (16) mit abnehmbaren Verbindungsteilen (33) versehen ist, damit die Kappe mit der Kapsel (13) fest verbunden ist, wenn die Spritze in ihrer ersten Stellung ist, und Abstützungen (39, 40) aufweist, um eine relativ begrenzte Verstellung des beweglichen Einspritzteiles und des Behälters zu ermöglichen, damit die vorgefüllte Spritze von ihrer ersten Stellung in ihre zweite Stellung gebracht wird, wobei die abnehmbaren Verbindungsteile (33) einen Ansatz (36) enthalten, der an der Kapsel 13 befestigt ist, wobei dieser Ansatz durch einen Bereich von geringerer Festigkeit mit der Kappe (16) verbunden ist.

2. Spritze gemäß Anspruch 1, dadurch gekennzeichnet, daß der Ansatz (36) an einem ringförmigen Rand (35) befestigt ist, der um die Kapsel (13) in der Nähe ihres Endes angeordnet ist, in den das offene Ende des Behälters eingeführt ist.

3. Spritze gemäß Anspruch 2, dadurch gekennzeichnet, daß der ringförmige Rand (35), der um die Kapsel (13) angeordnet ist, mindestens zwei seitliche Flügel (34) für das Halten der Spritze bildet.

4. Spritze gemäß Anspruch 2, dadurch gekennzeichnet, daß der Ansatz (36) mit Ultraschall an den ringförmigen Rand geschweißt ist.

5. Spritze gemäß Anspruch 1, dadurch gekennzeichnet, daß die Abstützungen mindestens eine Anschlag (40) enthalten, der so ausgebildet ist, um mit einem Rand der Kappe (16) zusammenzuwirken, und daß der Abstand (d) zwischen dem Anschlag und dem Rand der relativ begrenzten Verstellung des beweglichen Einspritzteils und des Behälters entspricht.

6. Spritze gemäß Anspruch 5, dadurch gekennzeichnet, daß der Anschlag eine Oberfläche (40) des ringförmigen Randes (35) der Kapsel (13) enthält und daß der Rand der Kappe (16), der mit diesem Anschlag zusammenwirkt, über einen Bereich von geringerer Festigkeit mit dem Anzatz (36) verbunden ist.

7. Spritze gemäß Anspruch 1, dadurch gekennzeichnet, daß der Ansatz eine allgemeine zylindro-konische Form aufweist, wobei der kleiner Durchmesser größer als der äußere Durchmesser der Kappe (16) ist, die eine allgemeine zylindrische Form hat.

8. Spritze gemäß Anspruch 7, dadurch gekennzeichnet, daß der Bereich von geringerer Festigkeit aus einem geschlossenen ringförmigen Teil (37) besteht, dessen Oberfläche sich seitlich zu dem äußeren Teil (16) der Kappe hin erstreckt.

9. Spritze gemäß Anspruch 7, dadurch gekennzeichnet, daß der Bereich von geringerer Festigkeit aus einer Reihe von Stegen, die zu dem äußeren Teil der Kappe hin seitlich aufgerichtet sind, gebildet ist.

10. Spritze gemäß Anspruch 1, dadurch gekennzeichnet, daß die Kappe mindestens eine Abstützung enthält, die innen angeordnet und so ausgebildet ist, um sich gegen den geschlossen Boden des Behälters (10) abzustützen.

11. Spritze gemäß Anspruch 1, die einen Nadelschutz (15) enthält, dadurch gekennzeichnet, daß dieser Nadelschutz mit Anschlägen versehen ist, die angeordnet sind, um als Begrenzung des Verstellweges für die Kontrolle der relativen Verstellung des Behälters (10) und des Verschlußteils (12) zu dienen, wobei diese Anschläge angeordnet sind, damit diese relative Verstellung der Einspritzung einer halben Dosis der Arznei entspricht.

## Claims

1. Prefilled syringe comprising an elongated reservoir (10) open at one of its ends (18) and provided with a constricted portion (17) adjacent to this end (18), this reservoir (10) containing a liquid (11) particularly a liquid medicine to be injected, an injection element movable with respect to this reservoir, this injection element comprising a blocking element (12) in elastomer at least partially engaged in the reservoir (10) in the constricted portion (17) and adapted to block the reservoir (10) when the syringe is in airs position said storage position, this blocking element being provided with a central conduit composed of an axial arm (26) and at least a transverse arm (27) to place in communication a needle (14) with the interior of the reservoir (10) so as to assure the evacuation of the liquid when the syringe is in a second position said injection position, the syringe beside comprising a capsule (13) integral with this blocking element (12) and mounted on the reservoir (10) such that one end slice of this reservoir adjacent to its opening (18) is engaged in this capsule (13) so as the relative displacement of the capsule (13) and of the reservoir (10) induces an equivalent displacement of the blocking element (12) in the reservoir (10) sufficient for driving back quantity of liquid medicine (11) sufficient to totally fill the central conduit and to appear in the form of a drop at the end of the needle (14), characterized in that the syringe is equipped with a cap (16) positioned on top of the closed end of the elongated reservoir and in that this cap (16) is provided with removable linkage means (33) to render this cap integral with the capsule (13) when the syringe is in its first position, and support means (39, 40) to allow for a relative limited displacement of the movable injection element and of the reservoir to bring the prefilled syringe from its first position into its second position, the said removable linkage means (33) comprising a base (36) attached to the capsule (13), this base being connected to the cap (16) by a zone of induced resistance.

2. Syringe according to claim 1, characterized in that the said base (36) is attached to an annular edge (35) provided around the capsule (13) adjacent to its end in which is engaged the open end of the reservoir.

3. Syringe according to claim 2, characterized in that the said annular edge (35) provided around the capsule (13) forms at least two lateral wings (34) for maintenance of the syringe.

4. Syringe according to claim 2, characterized in that the said base (36) is welded by ultrasound to the said annular edge (35).

5. Syringe according to claim 1, characterized in that the said support means comprise at least one abutment (40) connected to cooperate with a cap edge (16) and in that the distance (d) between this abutment and this edge is equal to the said relative limited displacement of the movable injection element and of the reservoir.

6. Syringe according to claim 5, characterized in that the said abutment comprises a surface (40) of the said annular edge (35) of the said capsule (13) and in that the said edge of the cap (16) which cooperates with this abutment is connected to the said base (36) by a zone or reduced resistance.

7. Syringe according to claim 1, characterized in that the base has a generally cylindrical conical shape whose smaller diameter is greater than the exterior diameter of the cap (16) of generally cylindrical shape.

8. Syringe according to claim 7, characterized in that the zone of reduced resistance is constituted by an annular closed element (37) whose surface extends laterally towards the exterior of the cap (16).

9. Syringe according to claim 7, characterized in that the zone of reduced resistance is constituted by a flap assembly extending towards the exterior of the cap.

10. Syringe according to claim 1, characterized in that the cap comprises at least one support element provided within and adapted to rest against the closed bottom of the reservoir (10).

11. Syringe according to claim 1, comprising a needle protector (15), characterized in that this needle protector is provided with abutments (41) adapted to serve to limit the course of movement adapted to control the relative displacement of the reservoir (10) and of the blocking element (12), these abutments being positioned such that the relative displacement corresponds to the injection of a half-dose of medicine.

Fig.1

Fig. 2

Fig. 3.